# EUROPEAN PATENT SPECIFICATION

(11) **EP 3 424 887 B1**
(45) Date of publication and mention of the grant of the patent: **16.09.2026**
(21) Application number: 18180292.7
(22) Date of filing: 28.06.2018
(51) Int. Cl.: C02F 11/04, C02F 1/68

(54) **USE OF AND ADDITIVE AND PROCESS FOR REDUCTION OF METHANE EMISSONS IN DIGESTATE**
VERWENDUNG EINES ADDITIVES UND VERFAHREN ZUR REDUKTION VON METHANEMISSIONEN IN GÄRRESTEN
UTILISATION D'UN ADDITIF ET PROCÉDÉ POUR LA RÉDUCTION DES ÉMISSIONS DE MÉTHANE DANS LE DIGESTAT

(30) Priority: 04.07.2017 SE 1750869
(43) Date of publication of application: 09.01.2019
(73) Proprietor: Tekniska Verken I Linköping AB, 581 15 Linköping (SE)
(72) Inventor: Nordell, Erik, 589 50 Linköping (SE); Moestedt, Jan, 602 24 Norrköping (SE)
(74) Representative: AWA Sweden AB

(56) References cited:
- EP-A1- 0 559 272
- KIM J O ET AL: "Enhancing continuous hydrogen gas production by the addition of nitrate into an anaerobic reactor", PROCESS BIOCHEMISTRY, ELSEVIER LTD, GB, vol. 41, no. 5, 1 May 2006 (2006-05-01), pages 1208 - 1212, XP027984138, ISSN: 1359-5113, [retrieved on 20060501]
- P HOEKSMA ET AL: "Monitoring methane and nitrous oxide reduction by manure treatment", 1 August 2012 (2012-08-01), XP055504208, Retrieved from the Internet <URL:http://edepot.wur.nl/238618> [retrieved on 20180904]
- REGUEIRO IRIA ET AL: "Acidification of raw and co-digested pig slurries with alum before mechanical separation reduces gaseous emission during storage of solid and liquid fractions", AGRICULTURE, ECOSYSTEMS AND ENVIRONMENT, ELSEVIER, AMSTERDAM, NL, vol. 227, 12 May 2016 (2016-05-12), pages 42 - 51, XP029546680, ISSN: 0167-8809, DOI: 10.1016/J.AGEE.2016.04.016
- F. MONLAU ET AL: "New opportunities for agricultural digestate valorization: current situation and perspectives", ENERGY & ENVIRONMENTAL SCIENCE, vol. 8, no. 9, 1 January 2015 (2015-01-01), Cambridge, pages 2600 - 2621, XP055325365, ISSN: 1754-5692, DOI: 10.1039/C5EE01633A

## Description

### Technical field

The present invention relates to the use of an additive for reduction of methane emission from digestate, i.e. the residue from anaerobic processes, and to a process for reducing methane emissions from digestate.

### Background

In recent years it has become more and more important to be able to reduce methane emission from digestate, mainly due to the ongoing climate changes largely impacted by gases such as methane. The digestate is defined by a liquid or a solid fraction that is left after treating organic material under anaerobic conditions, i.e. with the aim to produce biogas, containing methane. The origin of the digestate may be derived from many different types of anaerobic processes where different types of substrates may have been used. Such as anaerobic digestion of agriculture waste, food waste, slaughterhouse waste, sludge from waste water treatment plants etc. Usually the digestate is in liquid form (e.g. total solid concentration between 2-10%), but it may also be dewatered into a material having a high total solid content (e.g. total solid concentration between 20-35%).

The sludge residues from anaerobic processes, such as for instance biogas production, are conventionally placed in a post-digester or chamber to allow the anaerobic-process rate to decrease. After that the residues are often stored to be used as bio-fertilizers, it is stored either at the site of biogas production or at the farm where the fertilizer is to be used. The digestate or residuals may be in liquid form (untreated) or in solid form (dewatered).

These residues may be in different stages of degradation, which means that in some cased the degradation, and anaerobic processes may still continue after the sludge has been removed from the anaerobic digestion chamber. A study made in Sweden at different co-digestion plants concluded that one of the main sources of methane emissions at biogas plants was from the handling of digestate (AvfallSverige, 2012; Holmgren *et al.,* 2015). The methane emission from the sludge may be substantial, which is undesirable from a climate and environmental point of view since methane is 34 times worse than carbon dioxide due to Global Warming according to IPCCₜ₌₁₀₀ (IPCC, 2013).

The sludge residues may therefore be treated in different ways to reduce or inhibit the microbial activity to reduce the methane emissions. One such treatment is cooling the digestate or residual sludge to a temperature of around 15°C, at which point methane emissions usually stop. Another way is to aerate the digestate or sludge with air or oxygen, inhibiting of the microorganisms responsible for the anaerobic processes in the sludge. Cooling the digestate is a quite effective method, however it may also be quite costly depending on the amount of sludge residues to be cooled, and there is also a risk of formation of struvite (MgNH₄PO₄) and other precipitation due to the change in temperature. Aerating the sludge residues can also be effective, yet costly depending on the amount of residues to be treated.

In EP2457878A1 the use of different additives in the anaerobic digestion process are discussed, to improve the methane production in the digestion step. This document discloses that a nitrate salt is added in an amount of 1-4% of the added COD to improve the methane production. In Kim J. O. et al: "Enhancing continuous hydrogen gas production by the addition of nitrate into an anaerobic reactor", Process Biochemistry, ELSEVIER LTD, vol. 41, no. 5, 1 May 2006, pages 1208-1212, however, nitrate is added to suppress methane production in order to favour hydrogen production.

There is an imminent need to find a new way of reducing the methane production in sludge residues, which does not negatively influence the quality of the sludge residues, and which is comparatively economical.

### Summary

It is an object of the present disclosure, to provide an improved additive for reducing methane gas emissions from digestate and a process for reducing emissions from digestate.

The scope of the invention is defined by the appended independent claims. Embodiments are set forth in the appended dependent claims and in the following description.

According to a first aspect there is provided the use of an additive for reducing methane emissions in digestate, wherein the additive comprises a nitrate salt, wherein said additive is added to the digestate in an amount of at least 300 mg NO₃⁻/L of digestate, wherein additive is added to the digestate after said digestate has left digester or anaerobic digestion chamber.

This additive allows for a very efficient and cost effective reduction of emissions of methane gas from or in the digestate. The reduction is at least 10% of the methane emissions from a completely untreated digestate.

According to one embodiment the additive may added in an amount of at least 500 mg NO₃⁻/L, or at least 700 mg NO₃⁻/L or at least 1000 mg NO₃⁻/L of digestate.

The nitrate salt may be selected from the group comprising any one of sodium nitrate, potassium nitrate, calcium nitrate, ammonium nitrate or magnesium nitrate.

Nitrate salt such as ammonium nitrate or calcium nitrate will also contribute to the value and quality of the bio-fertilizer.

The additive may be in the form of a nitrate solution containing 0.001 to 80 weight-% dissolved nitrate salt.

The digestate to which the additive is added is anaerobic sludge residues from anaerobic digestion of any one of agriculture waste, food waste, slaughterhouse waste and sludge from waste water treatment plants.

The digestate may have a temperature of less than 37°C, or less than 33°C or less than 28°C, or less than 23°C when said additive is added.

The additive may be more effective at lower temperatures, i.e. temperature which are lower than the temperature that the anaerobic sludge usually has in the anaerobic digestion chamber. This temperature is conventionally around 38°C for mesophilic digestion and around 52°C for thermophilic digestion.

According to a second aspect there is provided a process for reducing methane emissions in digestate, wherein the digestate is anaerobic sludge residues from anaerobic digestion of any one of agriculture waste, food waste, slaughterhouse waste and sludge from waste water treatment plants, wherein said process comprises the step of adding an additive to said digestate, characterized in that said additive comprises a nitrate salt and wherein said nitrate salt is added in an amount of at least 300 mg NO₃⁻/L of digestate and wherein additive is added to the digestate after said digestate has left digester or anaerobic digestion chamber.

The nitrate salt may be added in an amount of at least 500 mg NO₃⁻/L, or at least 700 mg NO₃⁻/L or at least 1000 mg NO₃⁻/L of digestate.

The temperature of the digestate may be less than 37°C or less than 33°C or less than 28°C, or less than 23°C when said additive is added.

According to the process of the second aspect the methane emission from the digestate is reduced. The methane emission from the digestate may be reduced by at least 10%, or more preferably at least 25% or even more preferably at least 50%.

This means that the emission of methane gas is preferably considerably reduced as compared to an untreated digestate, i.e. a digestate to which no additive has been added, or to an untreated control. This does not mean that the methane emissions of an untreated portion of the digestate or a control portion are in fact measured when adding the nitrate salt additive.

The additive may be a nitrate salt selected from the group comprising any one of sodium nitrate, potassium nitrate, calcium nitrate, ammonium nitrate or magnesium nitrate.

### Brief description of drawings

Figs 1A-1C illustrate examples of different dosing alternatives for the additive.
Fig. 2 illustrate a first trial set up.
Fig. 3 illustrates the methane production in relation to the control reactor at different nitrate concentrations added, in the first trial.
Fig. 4 illustrates the nitrous oxide production in relation to the added nitrate salt, in the first trial.
Fig. 5 illustrates a second trial set up.

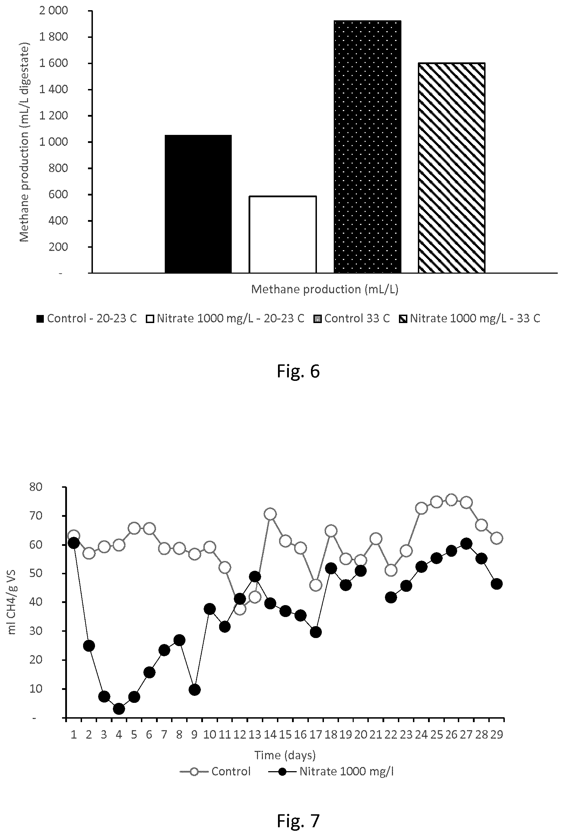

Fig. 6 illustrates the methane production in the second trial.
Fig. 7 illustrates the methane production over time in a third trial.

### Description of Embodiments

Different types of nitrate salt may be used as the additive in the present invention such as, but not limited to, calcium nitrate (Ca(NO₃)₂) or ammonium nitrate (NH₄NO₃). The active compound of the additive is the nitrate (NO₃⁻ ion) but this is dosed as a salt, i.e. with a cation such as for instance calcium or ammonium. However, the addition of ammonium nitrate may add additional value to a Bio-fertilizer since the content of ammonium increases. Furthermore the availability of ammonium nitrate makes it a cost efficient additive.

Another alternative is to produce the nitrate locally by circulating a small part of digestate to an aerated tank, to gain aerobic condition to oxidize ammonium to nitrate (NO₃⁻), and then pump the treated digestate to the main flow of digestate.

The additive, i.e. the nitrate salt may be dosed in solid form directly into said digestate.

Alternatively, the additive, or the nitrate salt, may be dissolved in an aqueous solution, such as water and dosed in a liquid form.

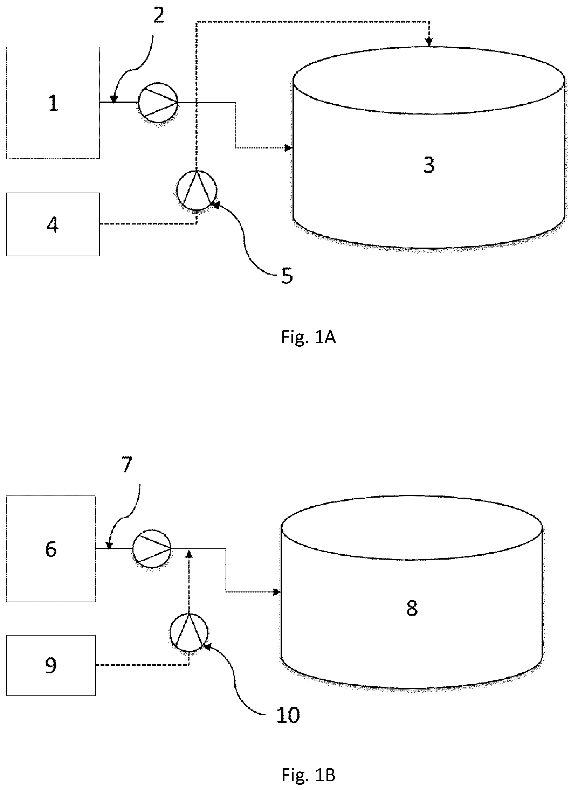

Different dosing alternatives are illustrated in Figs 1A to Fig 1C. As shown in Fig. 1A the digestate may be brought from an anaerobic digestion chamber 1, 6, through a transportation line or digestate pipe 2 into a storage tank 3. The additive may be dosed directly into the storage tank 3. As shown in Fig 1B, the additive 9 may also be added with a dosing pump 10 into the digestate pipe 7 and mixed into the digestate before it enters the storage tank 8.

The nitrate salt **is** dosed directly into the digestate after leaving the digester or anaerobic digestion chamber, for instance into a pipe 2, 7 between digester and digestate storage or directly into the storage tank 3, 8. If the additive is added as a liquid, the addition may be made through a dosing pump 5, 10. The solution comprising the additive may be kept in a storage or dosing tank 4, 9.

The storage tank 3, 8 for digestate can be located at the biogas plant and/or by the farmers. Normally a truck collects the digestate from the biogas plants storage tank and deliver it to the costumer's storage tank.

The nitrate salt may be added in any of those storage tanks or in the truck to prevent methane emissions from the digestate.

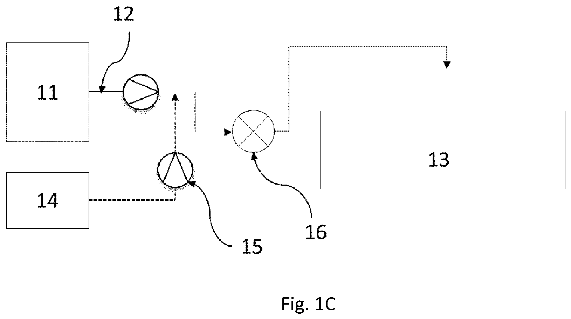

As shown in Fig. 1C, the additive can also be dosed prior to dewatering of digestate, into the digestate pipe 12 by a dosing pump 15 and/or in the dewatering equipment 16. The additive may be stored in a tank 14 if stored as a liquid. The additive (liquid or solid) may also be added directly to the dewatered digestate 13, even though mix-in of the additive may be challenging.

The additive is added in an amount of at least 300 mg NO₃⁻/L of digestate, or at least 500 mg NO₃⁻/L digestate.

The additive may be added in a range of from 300 mg NO₃⁻/L to 4 000 mg NO₃⁻ /L of digestate or even more preferable in a range of from 500 mg NO₃⁻/L to 2 000 mg NO₃⁻/L of digestate. An upper limit may be based on the cost of the nitrate salt in relation to the price of the subsequent bio-fertilizer, and also with regards taken to possible emissions of nitrous oxide (so called laughing gas). Nitrous oxide has recently been found to be detrimental to stratospheric ozone, comparable to the CFCs and nitrous oxide has a Global Warming Potential of 298 times worse than carbon dioxide according to IPCCₜ₌₁₀₀ (IPCC, 2013). A limitation and/or reduction of nitrous oxide emissions is therefore also required and must be considered when dosing nitrate into the digestate as these emissions are not collected, but enters into the atmosphere directly. The storage tank 3, 8 may be an open-air lagoon or open air tank. The addition of the nitrate salt may be made under aerobic conditions or close to aerobic conditions.

According to one alternative the temperature in the digestate to which the nitrate salt is added is preferably kept below that of the temperature in the anaerobic digestion chamber. The temperature of the digestate, when the nitrate is added is therefore preferably below the process temperature in the digester. The temperature of the digestate, when the nitrate is added is therefore preferably below 37°C, or below 33°C, or more preferably below 28°C, or even more preferably below 23°C.

### Trials

### Experiment 1

Digestate was collected on a daily basis from the full-scale biogas plant. The plant annually treats approximately 100 000 ton of organic waste such as food waste, slaughterhouse waste and industrial wastes. The plant produces biomethane with an annual production of around 110 GWh. The plant has three main digesters, or anaerobic digestion chambers, and one post-digester, with a total hydraulic retention time of 40-60 days. After digestion the digestate may be stored in a storage tank (in this case an open-air lagoon) with a volume of 4 000 m³. The volume in the storage tank varies depending on the in and out transport of digestate to/from the storage tank. Normally digestate is pumped to the storage tank continuously and digestate is collected by trucks 10 times a day, and transported to local farmers and their storage tanks. The digestate is used as bio-fertilizer and is a valuable product due to the high content of ammonium nitrogen (3 g NH₄-N/L).

Even though the hydraulic retention time is long in the anaerobic digestion chambers and the plant applies post-digestion, the methane emissions from the storage tank at the biogas plant and the storage tanks on farmers sites is an environmental problem (Nordell and Karlsson, 2011). The emissions depend on degree of degradation in the process, the temperature in the storage tank, the exposure for air and the storage time. Measurement performed by third-part at the site has shown that the emissions from the storage tank at the biogas plant is approximately 1% (or 110 000 kg CH₄/year) of the total biogas production at the plant (AvfallSverige, 2012; Holmgren *et al.,* 2015). The emissions of 1% corresponds to 3700 tons of CO₂-eq/year.

The aim of this experiment was to suppress the methane production and thus the emissions in digestate by adding nitrate salt. In this experiment different doses of nitrate salt were evaluated as well as different types of nitrate salt.

### Material and methods

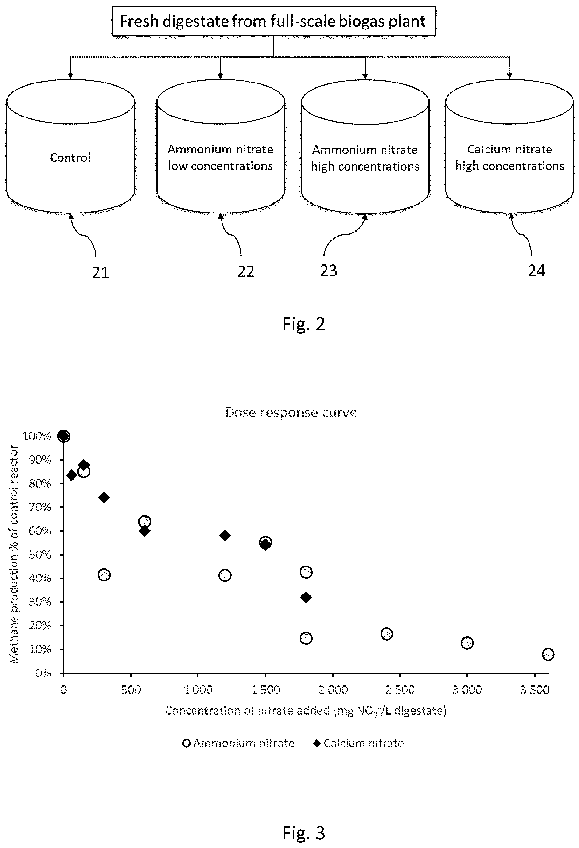

Four continuously stirred tank reactors 21, 22, 23, 24 (CSTR) in lab-scale (Nordell *et al.,* 2011) were used to evaluate the effect of nitrate salts addition to fresh digestate as shown in Fig. 2. Calcium nitrate (Ca(NO₃)₂) and ammonium nitrate (NH₄NO₃) were evaluated with respect to methane production. The reactor setup is shown in Fig. 1, where the 1st CSTR 21 was used as control reactor where only fresh digestate was added, in the 2nd CSTR 22 a low doses of ammonium nitrate was added, in the 3th CSTR 23 a high doses of ammonium nitrate was added and in the 4th CSTR 24 a low doses of calcium nitrate was added. Fresh digestate was collected from the full-scale plant on a daily basis. The reactors were feed once a day together with the additive, i.e. respective nitrate salt. The average hydraulic retention time was selected to be 10 days to mimic the conditions of the storage tank at the full-scale plant. The digesters were stirred with a mechanical stirrer and electrical motor with a rate of 100-200 rpm.

### Operational conditions

The CSTRs were fed once a day with approximately 24 hour's intervals. The digestion was performed at sub-mesophilic conditions (room temperature, approximately 20-23°C). Surplus reactor material was withdrawn before feeding to keep the effective volume constant. Samples were taken from the surplus digestate. Ammonium nitrate was added as powder directly into the digestate prior to feeding of the reactor and calcium nitrate was dissolved in a water solution and was added directly into the digestate prior to feeding.

**Table 1. Trial set up for CSTR 22, 23 and 24**

| **Days** | **Ammonium nitrate** | **Ammonium nitrate** | **Calcium nitrate** |
|---|---|---|---|
| | **(mg NO₃⁻/L)** | **(mg NO₃⁻/L)** | **(mg NO₃⁻/L)** |
| 0-13 | 0 | 0 | 0 |
| 14-28 | 60 | 1 800 | 60 |
| 29-38 | 150 | 2 400 | 150 |
| 39-49 | 300 | 3 000 | 300 |
| 50-59 | 600 | 3 600 | 600 |
| 60-69 | 1 200 | 4 200 | 1 200 |
| 70-79 | 1 500 | - | 1 500 |
| 80-90 | 1 800 | - | 1 800 |

The dosing was performed to reach a concentration of the nitrate of the ingoing digestate. When the dosage was increased according to table 1 (above), the nitrate was chock-dosed to achieve a steady-state concentration in the reactor according to the chosen dose.

### Analyzes

Volumetric gas production and methane concentration was measured online with Ritter milligas counter (MGC-10, Ritter, Germany) and gas sensor (BlueSens GmbH, Germany), respectively. The measurement data was exported to the program BacVis used for data acquisition and biogas analysis (BlueSens, Germany). All volumetric gas data presented here were converted to standard conditions at pressure 1.01325 bars and temperature 273.2 °K. Ammonium (NH₄-N) was analyzed according to FOSS Tecators application sub note 3502 with a Kjeltec 8200 (FOSS in Scandinavia, Sweden). Analyze of N₂O was performed with a Gas Chromatograph, Agilent 7890A (Agilent Technologies) and separation of the analytes was performed with 8ft Hayesep Q kolonn (1/8" Stainless steel, packed colon) (Agilent Technologies). The N₂O was detected by a µECD (63Ni). Nitrite and nitrate was analyzed on a Gallery Plus (Thermo Scientific Inc., USA) and was prior the analysis, filtrated through a 0.45 µm-filter; the method applied was based on ISO 15923-1:2013.

### Results

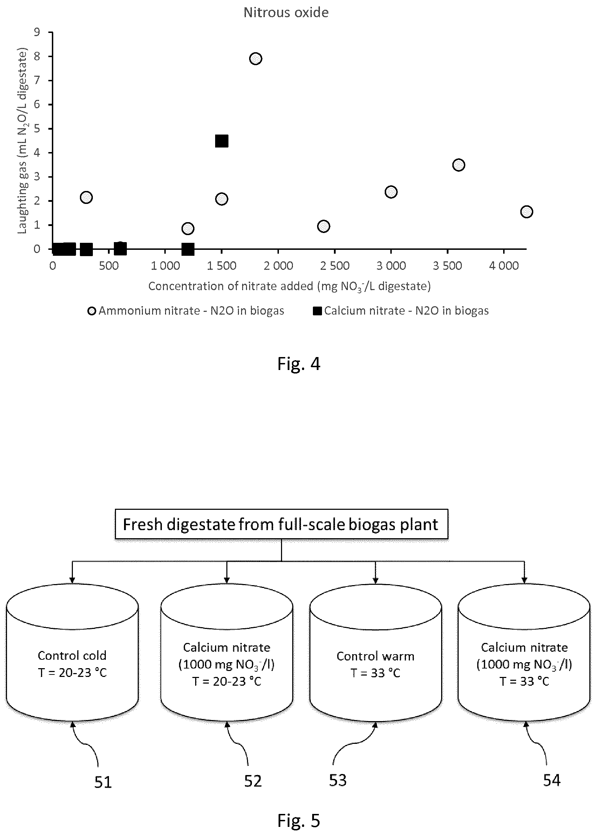

The results are shown in Fig. 3 and Fig. 4. The average methane production compared to the control reactor expressed as function to the dosed nitrate concentration in the ingoing digestate (Fig. 3). In Fig. 4 the production of nitrous oxide is shown with respect to concentration of nitrate added to the digestate.

### Main results and conclusions

There was a significant correlation between added nitrate concentration and decrease in methane production in the lab-scale CSTR-experiment. The inhibition/decrease in methane production was similar for both nitrate salts used, indicating that multiple nitrate salts may be used with the same effect/decrease of the methane production. Doses above 1 800 mg NO₃⁻/l resulted in a decreased methane production with 80-92% compared to the control reactor. N₂O could be detected in all samples but was low in general.

The addition of ammonium nitrate resulted in higher ammonium concentration in the digested sludge, which was expected. There were no differences in ammonium concentration in the calcium nitrate reactor compared to the control reactor, indicating that nitrate added is being converted into nitrogen gas rather than ammonium.

Even though nitrate was added, neither nitrate nor nitrite was detected in the treated digestate from the trial reactors, except for day 17 in the highconcentration reactor (1 800 mg NO₃⁻/L) when the nitrate concentration was 124 mg NO₃⁻/kg and nitrate 6.5 mg NO₂⁻/kg. The detection limit was 22 mg NO₃⁻ /kg and 3 mg NO₂⁻/kg, respectability.

### Experiment 2

The aim of the second experiment was to evaluate the effect of nitrate salts at different temperatures, by simulating a digestate storage tank in lab-scale conditions with CSTR reactors 51, 52, 53 and 54 as shown in Fig. 5.

### Operational conditions

The CSTRs were fed once a day with approximately 24 hour's intervals. The digestion was performed at sub-mesophilic conditions (room temperature, approximately 20-23 °C) in reactor 51 and 52; and at 33 °C (controlled with heating-jacket) in reactor 53 and 54. Surplus reactor material was withdrawn before feeding to keep the effective volume constant. Samples were taken from the surplus digestate. Calcium nitrate (1000 mg NO₃⁻/L inlet) was added as an additive (dissolved in water) directly into the digestate, prior to feeding reactor 52 and reactor 54. No chock-dose of nitrate was applied in this experiment.

The second experiment was carried out during a period of 41 days corresponding to 4 hydraulic retention times.

### Main results and conclusions

Lower temperature results in lower methane production and activity (well-known), and nitrate has a lower inhibitory effect at 33°C compared to 20-23°C.

At the lower temperature, the inhibition was -45% compared to -17% at 33°C.

In experiment 1 the inhibition was 60% at 1 200 mg NO₃⁻/L, at T = 20-23°C. In experiment 2 an initial concentration was not chock-dosed to the steady-state level, resulting in a possible adaptation, resulting in a lower inhibition grade than in experiment 1.

### Experiment 3

This was a repeat of the heated reactors (33 °C) of experiment 2, but performed at a later stage. Two CSTR reactors were used and the temperature was T = 33°C, acting control and one where nitrate was dosed. Digestate from the same biogas plant was used. The only difference was that ammonium nitrate was used, and the dose was chock-dosed in the beginning to reach an immediately steady-state concentration at 1 000 mg NO₃⁻/L in the reactor. The additive was added as powder directly when feeding the reactor with nitrate addition.

### Main results and conclusions

The initial inhibition was very high due to chock-dose. The last 10 days average decrease in methane production was -20% which is similar to experiment 2 where the inhibition was -17% at the same dose, and the same temperature (33 °C).

### References

AvfallSverige, 2012. Sammanställning av mätningar inom Frivilligt åtagande 2007-2012**.**

Holmgren, M.A., Nörregard Hanssen, M., Reinelt, T., Westerkamp, T., Jörgensen, L., Scheutz, C., Delre, A., 2015. Measurements of methane emissions from biogas production. 2014. doi: 10.13140/RG.2.1.1007.4087.

IPCC, 2013. Working Group I Contribution to the IPCC Fifth Assessment Report. Climate Change 2013: The Physical Science Basis - Summary for Policymakers**.**

Nordell, E., Karlsson, M., 2011. Post digestion of biogas production residues at mid-range mesophilic temperature. International IWA-Symposium on Anaerobic Digestion of Solid Waste and Energy Crops, Vienna, Austria, August 28th - September 1st.

Nordell, E., Moestedt, J., Karlsson, M., inventor; Tekniska verken i Linköping AB (publ.), assigneee, 2011. Biogas Producing Laboratory Reactor. SE patent SE Patent Application no 1150954-4.

YARA International ASA, assignee published 30.05.2012. Anaerobic sludge treatment processes. EP2457878A1

## Claims

1. Use of an additive for reducing methane emissions in digestate,
wherein the digestate is anaerobic sludge residues from anaerobic digestion of any one of agriculture waste, food waste, slaughterhouse waste and sludge from waste water treatment plants,
wherein the additive comprises a nitrate salt,
**characterized in that**
said additive is added to the digestate in an amount of at least 300 mg NO₃⁻/L of digestate, and wherein additive is added to the digestate after said digestate has left digester or anaerobic digestion chamber.

2. The use as claimed in claim 1, wherein the additive is added in an amount of at least 500 mg NO₃⁻/L, or at least 700 mg NO₃⁻/L or at least 1000 mg NO₃⁻/L of digestate.

3. The use as claimed in any one of claims 1 or 2, wherein said nitrate salt is selected from the group comprising any one of sodium nitrate, potassium nitrate, calcium nitrate, ammonium nitrate or magnesium nitrate.

4. The use as claimed in any one of claims 1 or 3, wherein said additive is a nitrate solution containing 0.001 to 80 weight-% dissolved nitrate salt.

5. The use as claimed in any one of the preceding claims, wherein said digestate has a temperature of less than 37°C, or less than 33°C or less than 28°C, or less than 23°C when said additive is added.

6. A process for reducing methane emissions in digestate, wherein said process comprises the step of adding an additive to said digestate,
wherein the digestate is anaerobic sludge residues from anaerobic digestion of any one of agriculture waste, food waste, slaughterhouse waste and sludge from waste water treatment plants, **characterized in that** said additive comprises a nitrate salt and wherein said nitrate salt is added in an amount of at least 300 mg NO₃⁻/L of digestate, and wherein additive is added to the digestate after said digestate has left digester or anaerobic digestion chamber.

7. The process as claimed in claim 6, wherein said nitrate salt is added in an amount of at least 500 mg NO₃⁻/L, or at least 700 mg NO₃⁻/L or at least 1000 mg NO₃⁻/L of digestate.

8. The process for reducing methane emission as claimed in any one of claim 6 or 7, wherein the temperature of the digestate is less than 37°C or less than 33°C or less than 28°C, or less than 23°C when said additive is added.

9. The process as claimed in any one of claims 6 to 8, wherein said additive is a nitrate salt selected from the group comprising any one of sodium nitrate, potassium nitrate, calcium nitrate, ammonium nitrate or magnesium nitrate.

## Patentansprüche

1. Verwendung eines Additivs zum Verringern der Methanemissionen in Gärgut,
wobei das Gärgut anaerober Schlammrückstand aus anaerober Vergärung von einem von landwirtschaftlichem Abfall, Lebensmittelabfall, Schlachthofabfall und Schlamm aus Abwasserbehandlungsanlagen ist,
wobei das Additiv ein Nitratsalz umfasst,
**dadurch gekennzeichnet, dass**
das Additiv in einer Menge von wenigstens 300 mg NO₃⁻/l Gärgut zu dem Gärgut zugegeben wird, und wobei das Additiv dem Gärgut zugegeben wird, nachdem das Gärgut den Gärbehälter oder die anaerobe Gärkammer verlassen hat.

2. Verwendung nach Anspruch 1, wobei das Additiv in einer Menge von wenigstens 500 mg NO₃⁻/l oder wenigstens 700 mg NO₃⁻/l oder wenigstens 1000 mg NO₃⁻/l Gärgut zugegeben wird.

3. Verwendung nach einem der Ansprüche 1 oder 2, wobei das Nitratsalz ausgewählt ist aus der Gruppe umfassend eines von Natriumnitrat, Kaliumnitrat, Calciumnitrat, Ammoniumnitrat und Magnesiumnitrat.

4. Verwendung nach einem der Ansprüche 1 oder 3, wobei das Additiv eine Nitratlösung ist, die 0,001 bis 80 Gew.-% gelöstes Nitratsalz enthält.

5. Verwendung nach einem der vorstehenden Ansprüche, wobei das Gärgut eine Temperatur von weniger als 37 °C oder weniger als 33 °C oder weniger als 28 °C oder weniger als 23 °C aufweist, wenn das Additiv zugegeben wird.

6. Verfahren zum Verringern von Methanemissionen in Gärgut, wobei das Verfahren den Schritt des Zugebens eines Additivs zu dem Gärgut umfasst,
wobei das Gärgut anaerober Schlammrückstand aus anaerober Vergärung von einem von landwirtschaftlichem Abfall, Lebensmittelabfall, Schlachthofabfall und Schlamm aus Abwasserbehandlungsanlagen ist, **dadurch gekennzeichnet, dass** das Additiv ein Nitratsalz umfasst und wobei das Nitratsalz in einer Menge von wenigstens 300 mg NO₃⁻/l Gärgut zugegeben wird und wobei das Additiv dem Gärgut zugegeben wird, nachdem das Gärgut den Gärbehälter oder die anaerobe Gärkammer verlassen hat.

7. Verfahren nach Anspruch 6, wobei das Nitratsalz in einer Menge von wenigstens 500 mg NO₃⁻/l oder wenigstens 700 mg NO₃⁻/l oder wenigstens 1000 mg NO₃⁻/l Gärgut zugegeben wird.

8. Verfahren zum Verringern der Methanemission nach einem der Ansprüche 6 oder 7, wobei die Temperatur des Gärguts weniger als 37° C oder weniger als 33 °C oder weniger als 28 °C oder weniger als 23 °C beträgt, wenn das Additiv zugegeben wird.

9. Verfahren nach einem der Ansprüche 6 bis 8, wobei das Additiv ein Nitratsalz ausgewählt aus der Gruppe umfassend eines von Natriumnitrat, Kaliumnitrat, Calciumnitrat, Ammoniumnitrat und Magnesiumnitrat ist.

## Revendications

1. Utilisation d'un additif pour réduire les émissions de méthane dans un digestat,
dans laquelle le digestat est des résidus de boue anaérobie provenant de la digestion anaérobie de l'un quelconque parmi des déchets agricoles, des déchets alimentaires, des déchets d'abattoirs et des boues de stations d'épuration des eaux usées,
dans laquelle l'additif comprend un sel de nitrate,
**caractérisée en ce que**
ledit additif est ajouté au digestat en une quantité d'au moins 300 mg NO₃⁻/L de digestat, et dans laquelle l'additif est ajouté au digestat après que ledit digestat a quitté le digesteur ou la chambre de digestion anaérobie.

2. Utilisation selon la revendication 1, dans laquelle l'additif est ajouté en une quantité d'au moins 500 mg de NO₃⁻/L, ou au moins 700 mg de NO₃⁻/L ou au moins 1 000 mg de NO₃⁻/L de digestat.

3. Utilisation selon l'une quelconque des revendications 1 ou 2, dans laquelle ledit sel de nitrate est choisi dans le groupe comprenant l'un quelconque parmi le nitrate de sodium, le nitrate de potassium, le nitrate de calcium, le nitrate d'ammonium ou le nitrate de magnésium.

4. Utilisation selon l'une quelconque des revendications 1 ou 3, dans laquelle ledit additif est une solution de nitrate contenant 0,001 à 80 % en poids de sel de nitrate dissous.

5. Utilisation selon l'une quelconque des revendications précédentes, dans laquelle ledit digestat a une température inférieure à 37 °C, ou inférieure à 33 °C ou inférieure à 28 °C, ou inférieure à 23 °C lorsque ledit additif est ajouté.

6. Procédé de réduction des émissions de méthane dans un digestat, dans lequel ledit procédé comprend l'étape d'ajout d'un additif audit digestat,
dans lequel le digestat est des résidus de boue anaérobie provenant de la digestion anaérobie de l'un quelconque parmi des déchets agricoles, des déchets alimentaires, des déchets d'abattoirs et des boues de stations d'épuration des eaux usées, **caractérisé en ce que** ledit additif comprend un sel de nitrate et dans lequel ledit sel de nitrate est ajouté en une quantité d'au moins 300 mg NO₃⁻/L de digestat, et dans lequel l'additif est ajouté au digestat après que ledit digestat a quitté le digesteur ou la chambre de digestion anaérobie.

7. Procédé selon la revendication 6, dans lequel ledit sel de nitrate est ajouté en une quantité d'au moins 500 mg de NO₃⁻/L, ou au moins 700 mg de NO₃⁻/L ou au moins 1 000 mg de NO₃⁻/L de digestat.

8. Procédé de réduction des émissions de méthane selon l'une quelconque des revendications 6 ou 7, dans lequel la température du digestat est inférieure à 37 °C ou inférieure à 33 °C ou inférieure à 28 °C, ou inférieure à 23 °C lorsque ledit additif est ajouté.

9. Procédé selon l'une quelconque des revendications 6 à 8, dans lequel ledit additif est un sel de nitrate choisi dans le groupe comprenant l'un quelconque parmi le nitrate de sodium, le nitrate de potassium, le nitrate de calcium, le nitrate d'ammonium ou le nitrate de magnésium.
